# EUROPEAN PATENT SPECIFICATION

(11) **EP 4 417 250 B1**
(45) Date of publication and mention of the grant of the patent: **04.02.2026**
(21) Application number: 24157689.1
(22) Date of filing: 14.02.2024
(51) Int. Cl.: A61N 5/06

(54) **PHOTOTHERAPY MASK**
MASKE FÜR DIE PHOTOTHERAPIE
MASQUE DE PHOTOTHÉRAPIE

(30) Priority: 14.02.2023 CN 202320260536 U; 10.03.2023 GB 202303566
(43) Date of publication of application: 21.08.2024
(73) Proprietor: MZ Skin Limited, London W1G 3NB (GB)
(72) Inventor: LIANG, Hongshan, Zhejiang Province P.R.C 315400 (CN)
(74) Representative: Slingsby Partners LLP

(56) References cited:
- CN-U- 210 750 922
- KR-A- 20180 035 629
- KR-A- 20220 065 598
- US-A1- 2018 352 936
- US-A1- 2021 370 090
- US-A1- 2022 080 221
- US-A1- 2023 033 993

## Description

### TECHNICAL FIELD

This application relates to a phototherapy mask.

### BACKGROUND

Face masks are known in which a posterior surface of the mask is provided with light emitters. When the mask is positioned against the face of a wearer, the light emitters can be activated. It is believed that this can provide a phototherapeutic effect, for example by improving skin condition, boosting collagen production and inhibiting disorders of the skin. Face masks may be rigid, semi-rigid, or flexible. Rigid face masks do not generally conform well to the user's face compared with flexible face masks. However, highly flexible face masks can be difficult to use and tend to be dislodged from the face easily. Semi-rigid face masks can be shaped to naturally conform to the user's face whilst being flexible enough for the user to adjust the mask to better fit their face. Semi-rigid face masks tend to be more complicated and costly to manufacture.

It is desirable to maximise the amount of skin underneath the mask that is exposed to radiation from the light emitters when the mask is in use. The more skin that can be irradiated by the face mask, the greater the expected therapeutic effect on the user's face. One way of increasing the proportion of the user's face beneath the mask that is exposed to light radiation may be to increase the density of light emitters in the face mask. However, this increases the cost of producing the mask and may result in some areas of skin being subject to excessive radiation.

Some areas of the face are particularly difficult to provide radiation to using a face mask that is semi-rigid. For example, areas of skin around the wearer's eyes, lips, and nose can be difficult to radiate because conventional face masks contain apertures or shields around these areas for safety and practicality. Apertures and shields such as these reduce the therapeutic effects of the mask, particularly because areas of skin around the eyes, lips and nose can be common problem areas for certain skin conditions (e.g., wrinkles, acne). As another example, areas of skin around the edge of the user's face, such as near the user's hairline, often do not get sufficient radiation due to ill-fitting masks. This further reduces the therapeutic effects of the face mask.

There is a need for an improved form of face mask. There is a need to increase the ease with which semi-rigid face masks can be manufactured. There is a need to increase the proportion of the wearer's face exposed to light radiation. There is a need to reduce costs of manufacturing the semi-rigid face mask, without significantly reducing the quality and effectiveness of the end product.

CN 210750922U relates to a beauty mask wherein the transparent inner shell, the flexible LED lamp panel and the non-transparent outer shell are sequentially arranged in a stacked mode from inside to outside.

US 2021/0370090 discloses systems, devices, and related methods for phototherapeutic treatment of skin, and more particularly phototherapeutic treatments for skin conditioning and/or the treatment of skin wrinkles.

US 2022/0080221 discloses a therapeutic face mask having a plurality of light emitting diodes.

US 2023/033993 discloses a phototherapy mask for phototherapy treatment of a user. US 2018/0352936 discloses a mask device for facial skin care.

KR 20180035629 discloses a mask using a light emitting element for improving skin. KR 20220065598 discloses an LED mask capable of implementing various care modes, facilitating attachment and detachment of the LED mask, and preventing LED light from entering user's eyes.

### SUMMARY OF INVENTION

The invention relates to a phototherapy mask and to a corresponding method for forming the same as defined in the appended claims. Embodiments, examples or aspects in the following disclosure which do not fall under the scope of the claims are presented for illustration purposes only and do not form part of the invention.

According to one aspect there is provided a phototherapy mask comprising:
a backing layer having a rear face and a front face and one or more lateral faces extending between the front face and the rear face;
a translucent layer disposed on the rear face of the backing layer, the translucent layer covering the entire rear face of the backing layer; and
a plurality of light emitters sandwiched between the rear face of the backing layer and the translucent layer;
wherein the backing layer and the translucent layer together form a flexible structure that adopts a form that is concave to the rear of the mask.

The mask may comprise a nose hole and a mouth hole. The translucent layer extends to the edges of the holes such that light from the light emitters can be emitted laterally from the translucent layer at the nose and mouth holes.

The mask comprises two eye holes.

The backing layer comprises a collar around each eye hole. Each collar extends rearwardly.

Each collar has an interior lateral surface that faces the respective eye hole and an exterior lateral surface. The translucent layer covers the exterior lateral surface of each collar.

The translucent layer extends to cover the rear facing portion of each collar.

The translucent layer has a rear face and the shape of the rear face of the translucent layer conforms to the shape of the rear face of the backing layer.

The translucent layer has a rear face and the shape of the rear face of the translucent layer conforms to the shape of the exterior lateral surface of each collar.

The backing layer may define a step along the periphery of the rear face of the backing layer. The translucent layer may define a locating feature shaped to mate with the step.

The translucent layer may cover the peripheral lateral face of the backing layer.

The mask may comprise a central plateau and cheek regions extending rearwardly and laterally from the central plateau.

The mask may comprise attachment structures at the rear of the cheek regions and one or more flexible straps attached to the attachment structures, wherein the attachment structures may be constituted by both the backing layer and translucent layer.

The light emitters may be capable of emitting one or both of infrared and ultraviolet light. The light emitters may be dispersed substantially uniformly over the area of the mask.

The backing layer and the translucent layer may together form a flexible structure that adopts a human facial form.

The Shore A hardness of the translucent layer may be in the range from 20 to 40. The Shore A hardness of the backing layer may be in the range from 30 to 50.

By implementing the above aspect, the therapeutic effect of the mask can be improved.

According to another aspect there is provided a method for forming a phototherapy mask, comprising:
forming a backing layer of a polymer material, the backing layer having a rear face and a front face and one or more lateral faces extending between the front face and the rear face;
disposing a plurality of light emitters over the rear face of the backing layer;
forming, separately to the formation of the backing layer, a translucent layer of a polymer material for covering the entire rear face of the backing layer; and
attaching the translucent layer to the rear face of the backing layer so as to sandwich the light emitters between the translucent layer and backing layer.

The backing layer may define a step around the periphery of the rear face of the backing layer. The translucent layer may define a locating feature shaped to mate with the step. Attaching the translucent layer to the rear face of the backing layer may comprise mating the locating feature with the step.

The backing layer and translucent layer each comprise two eye holes and collars extending rearwardly around each eye hole, wherein attaching the translucent layer to the rear face of the backing layer comprises fitting each collar of the translucent layer around the corresponding collar of the backing layer.

The backing layer and the translucent layer may each comprise attachment structures at the peripheral edges of the mask for attaching to flexible straps, wherein attaching the translucent layer to the rear face of the backing layer may comprise attaching one of the flexible straps to a respective attachment structure in each layer.

The translucent layer may be formed as one continuous piece of the polymer material.

Attaching the translucent layer to the rear face of the backing layer may comprise applying an adhesive to one or both of the rear face of the backing layer and the translucent layer. The method may further comprise pressing the translucent layer onto the backing layer to force the adhesive to fill the space between the backing layer and translucent layer.

### BRIEF DESCRIPTION OF DRAWINGS

The present invention will be described by way of example with reference to the drawings.
Figure 1 shows the posterior of a face mask.
Figure 2 shows the front of the face mask.
Figure 3 shows face mask of figure 1 from below, with the posterior side to the bottom of the figure.
Figure 4 shows a stereoscopic view of the mask, with the front of the mask to the left of the figure.
Figure 5 shows a cross-section through a part of the face mask around the eye area.
Figure 6 shows a simplified example of a collar viewed from the side of the mask.

### DETAILED DESCRIPTION

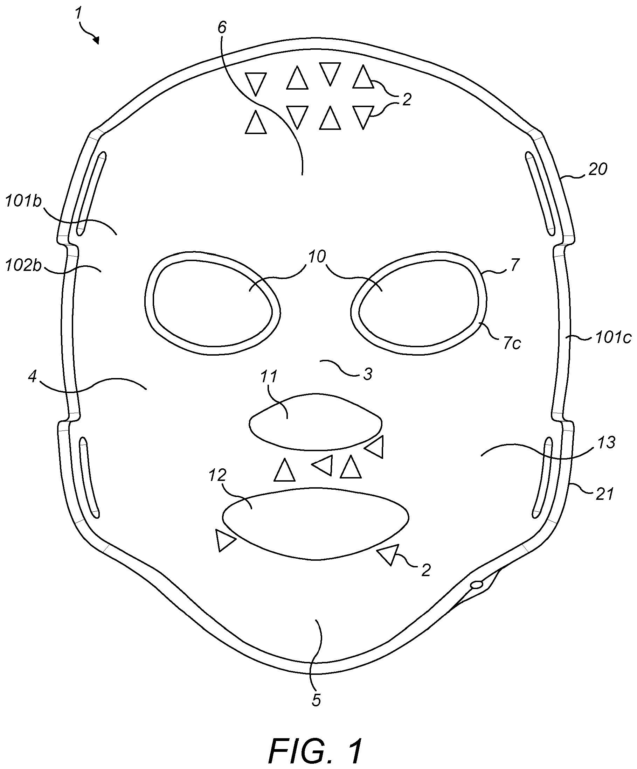

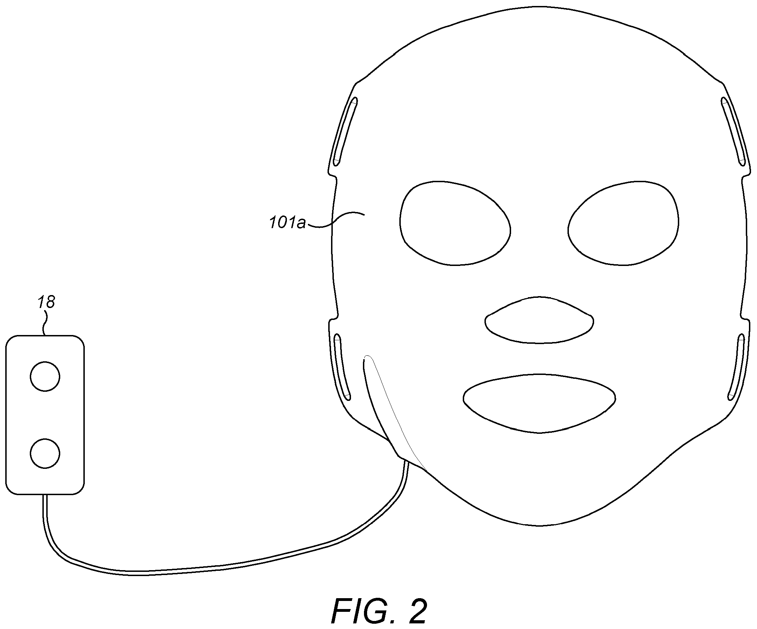

Figure 1 shows the rear face of a phototherapy mask 1. The rear face of the mask, equivalently referred to herein as the posterior face or posterior of the mask, faces the wearer when the mask is being worn. The face mask 1 is made of a backing layer 101 and a translucent layer 102. The backing layer has a front face 101a, shown in figure 2, and a rear face 101b, shown in figure 1. The translucent layer is disposed on the rear face of the backing layer. Together, the backing layer and the translucent layer form a flexible, resilient face mask. Light emitters 2 are sandwiched between the rear face of the backing layer and the translucent layer. The light emitters may be disposed in a uniform pattern over the rear face of the backing layer. Only a few light emitters are shown in figure 1 for clarity. Light from the light emitters diffuses through the translucent layer to reach the skin of the wearer. The translucent layer covers the entire rear face of the backing layer. In other words, the translucent layer extends over the whole of the rear face of the backing layer, all the way to the edges of the backing layer. Having the translucent layer extend over the entire rear face of the backing layer can increase the diffusion of light from the light emitters to the skin of the wearer. For example, the skin opposite parts of the face mask where there is a reduced density of light emitters can still be irradiated due to the diffusion of light through the translucent layer. Thus, areas of skin near the peripheral edges of the face mask (such as the hairline) and areas of skin around holes in the face mask (such as around the eyes, nose, or mouth) may be irradiated by the light emitters via diffusion of light through the translucent layer. This may improve the therapeutic effect of the mask, without having to significantly increase the density of light emitters in the mask.

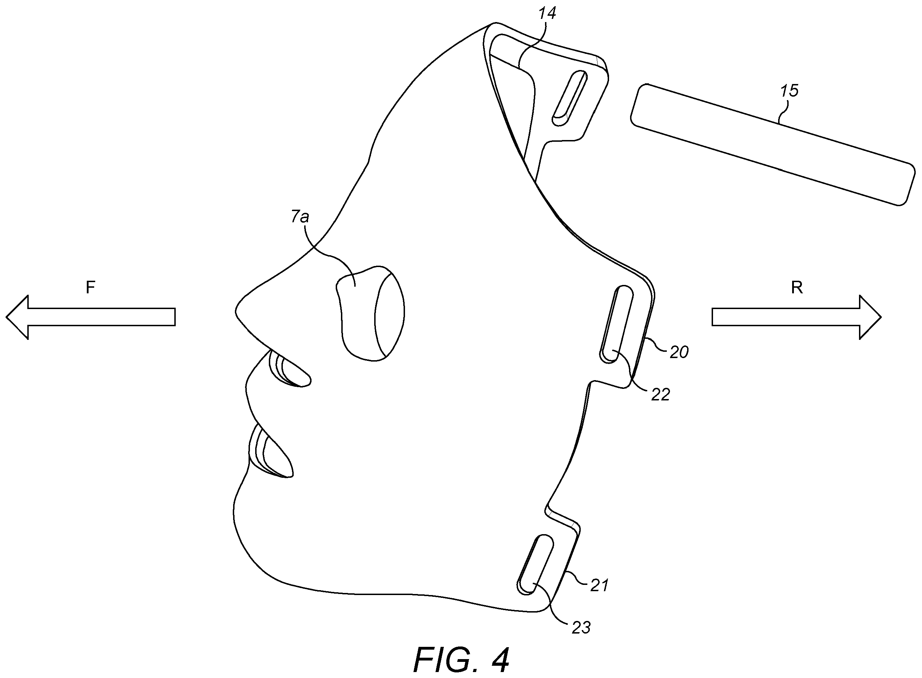

In more detail, figure 1 shows the face mask from the rear. The rear of the face mask refers to the side of the mask that faces the user when the face mask is worn. References herein to "rear facing", "rearwardly", or "in a rear direction", refer to the side of the mask that faces the user when worn (e.g., the portion of the mask that is visible when viewing the face mask from the rear). For example, figure 4 shows an arrow R that represents the rear facing direction with respect to the mask. The arrow F represents the front facing direction with respect to the mask. The front of the mask is the part of the mask that faces away from the user when worn (e.g., the portion of the mask that is visible when viewing the face mask from the front). The rear side of the mask may be generally concave between one extreme edge and the other and/or between the extreme top and the extreme bottom. This can provide a dished shape to the mask which can enable it to envelop a wearer's face.

The backing layer 101 comprises a front face 101a and a rear face 101b. The front face of the backing layer is visible from the front of the mask (e.g., see figure 2). The rear face of the backing layer is visible in figure 1. The backing layer comprises one or more lateral faces 101c that extend between the rear face and the front face. The peripheral lateral face of the backing layer 101c is indicated in figure 1. Holes in the backing layer such as nose hole 11 introduce other lateral faces of the backing layer, not shown in figure 1. Those lateral faces form rims of the holes transverse to the front and rear faces of the mask.

A translucent layer 102 is disposed on the rear face of the backing layer. The translucent layer covers the entire rear face of the backing layer. For example, the translucent layer covers all the surface of the rear face of the backing layer. In one example the translucent layer may be configured so that none of the rear face of the backing layer is uncovered by the translucent layer. Since the rear of the mask will be expected to touch the wearer, when the whole of the rear of the backing layer is covered by the translucent layer, the backing layer may be formed of a material that has a lower level of biocompatibility than might otherwise be required.

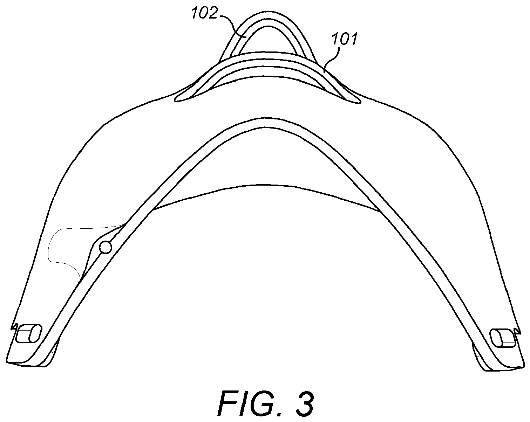

The translucent layer comprises a front face 102a and a rear face 102b. The front face 102a of the translucent layer (not shown) contacts the rear face of the backing layer. The rear face 102b of the translucent layer is visible in figure 1. The rear face 101b of the backing layer is visible in figure 1 through the translucent layer. The two layers can be seen in figure 3 around the nose and mouth holes. The rear face of the translucent layer 102b contacts the wearer's skin when the mask is worn.

A plurality of light emitters 2 are disposed on the rear face of the backing layer. The light emitters are sandwiched between the rear face of the backing layer and the front face of the translucent layer. The translucent layer may protect the light emitters. The translucent layer diffuses light emitted by the light emitters so as to improve its uniformity and range, as will be described in more detail below.

Together, the backing layer and translucent layer form a flexible structure that adopts a form that is concave to the rear of the mask. The mask may be semi-rigid. The mask may adopt (e.g., self-supportingly) a concave-downwards shape when laid with its rear on and facing a flat horizontal surface. The mask may adopt a concave-upwards shape when laid with its front on and facing a flat horizontal surface. The mask adopting a concave shape may mean that the overall shape of the mask is concave. Parts of the mask may be convex within that overall concave shape. The face mask has a pre-formed three-dimensional shape which resembles the contours of a human face.

The face mask may comprise a nose hole 11. The face mask may comprise a mouth hole 12. The face mask may comprise one or more eye holes 10. Typically, the face mask comprises two eye holes. The location of the eye, nose and mouth holes of the face mask (equivalently referred to herein as apertures) correspond, when the mask is in place on a wearer's face, to the eyes (apertures 10), the base of the nose (aperture 11) and the mouth (aperture 12) of the wearer.

As mentioned above, the translucent layer covers the entire rear face of the backing layer. When the backing layer comprises holes such as nose and mouth holes, the translucent layer extends to the edges of the holes. For example, in figure 3, both the translucent layer 102 and the backing layer 101 can be seen around the edge of nose hole and mouth hole. The translucent layer shown in figure 3 extends up to the edges of both holes. This can provide several benefits. Firstly, as the translucent layer extends up to the edges of the nose and mouth holes, light can be diffused through the translucent layer to irradiate the skin around the nose and mouth holes. Light can be emitted laterally from the translucent layer at the nose and mouth holes. In other words, light from the light emitters can diffuse through the translucent layer around the edges of the holes in a lateral (e.g., sideways) direction when viewing the mask from the rear or front. Secondly, as translucent layer can protect the light emitters from damage and acts to sandwich the light emitters between the backing layer, more light emitters may be positioned around the edges of the holes in the mask. This allows for better irradiation of the skin around the holes. The areas of skin around the nose, mouth, and eyes often need the most targeting for therapeutic purposes. However, they are particularly hard to radiate due to the apertures in the mask. With the arrangement described above, more light can be irradiated to the difficult areas around the nose and mouth. These effects apply to any hole in the face mask, not just the nose or mouth holes.

The face mask shown in the figures comprises two eye holes 10. Eye holes in light emitting face masks reduce the amount of light from the light emitters that can enter the eyes, and allow the user to see whilst wearing the mask, improving the safety and comfort of the mask. Due to the eye holes, the skin around the eye holes does not usually benefit from the therapeutic effects of the mask. However, the skin around the eye area is prone to wrinkles and other skin conditions. So, it is desirable to irradiate the skin around the eye in a safe manner to improve the therapeutic effects of the face mask.

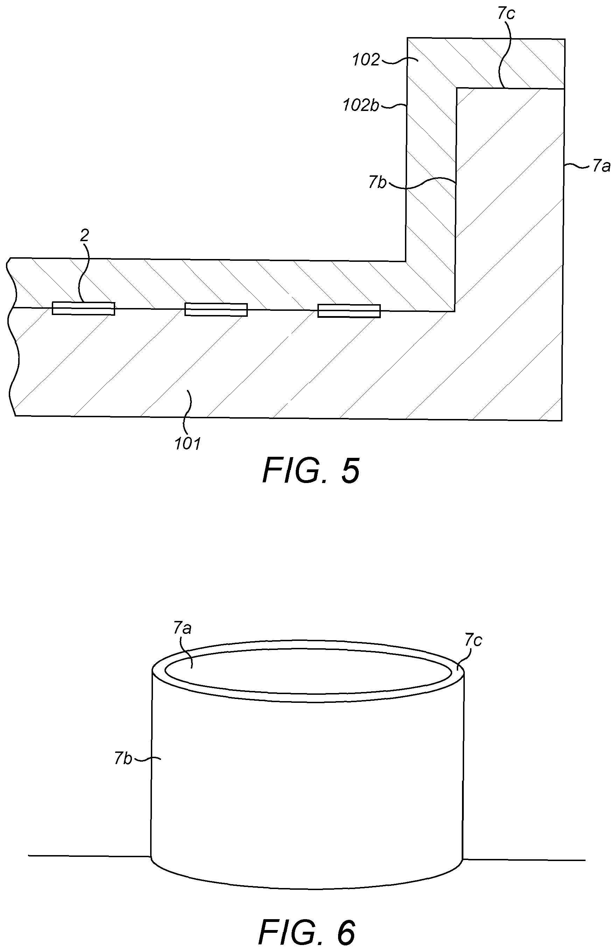

The backing layer may comprise a collar 7 around each eye hole. The collar can be seen from the front of the mask in figure 4. An example of a collar is shown in figure 6. The term "collar" may equivalently be referred to as a surrounding wall or border. The collars 7 around each eye hole extend rearwardly. In other words, the walls surrounding each eye hole (e.g., the collars) have sides that extend in the rear direction of the mask. When the mask is worn, the collars (e.g., walls) extend rearwardly towards the eyes of the user. The collars may make contact with the wearer's skin around their eyes to inhibit light generated by the mask from entering the wearer's eyes. The backing layer may be opaque. This may prevent light from the light emitters diffusing through the backing layer to reach the wearer's eyes. Each collar has an interior lateral surface 7a. The interior lateral surface of the collar faces the respective eye hole that the collar surrounds. The interior lateral surface of one collar can be seen in figure 4. The collar comprises an exterior lateral surface 7b. The exterior lateral surface may be parallel to the interior lateral surface. The exterior lateral surface of each collar faces away from the respective eye hole. The lateral surfaces of each collar are not visible in figure 1. The lateral surface of the collar refers to the surface of the collar that faces the lateral (e.g., sideways) direction with respect to the rear face of the backing layer. The lateral surface of each collar (7a, 7b) may be roughly perpendicular to the plane of the backing layer. For the avoidance of doubt, figure 6 shows a simplified example of a collar viewed from the side of the mask, where the bottom of the collar connects to the rear face of the backing layer. As can be seen in figure 6, the lateral surfaces of the collar are the sides of the collar that are roughly perpendicular to the plane of the backing layer (at the bottom of the figure). The exterior lateral surface of the collar may be equivalently referred to as the outer surface of the collar.

The translucent layer extends to cover the exterior lateral surface (e.g. the outer surface) of each collar. This can increase the diffusion of light to areas of skin around the edges of eye within the bounds of the face mask.

Each collar 7 comprises a rear facing portion 7c. The rear facing portion of the collar extends between the interior lateral surface and the exterior lateral surface of the collar. The rear facing portion faces the rear of the mask. In other words, the rear facing portion of the collar is the portion of the collar that is visible from the rear of the mask. The rear facing portion of the collar may be equivalently referred to as the top face of the collar, where the bottom face of the collar contacts the rear face of the backing layer (e.g., see figure 6). The rear facing portion of the collar may be considered the rim of the respective eye hole.

Figure 5 shows a cross section of a portion of the mask near one of the eye holes. The eye hole is to the right of the portion of the mask shown in figure 5. As shown in figure 5, the translucent layer may extend to cover the rear facing portion 7c of the collar. in other words, the translucent layer covers the rear portion of the collar such that, when worn, the rear facing portion of the collar does not come into contact with the skin of the user. This prevents the backing layer (e.g., the rear facing portion of the collar) from contacting the wearer's skin around the eye. The skin around the eye area can be particularly sensitive, so it is important that the material touching the eye area is suitable for sensitive skin. The translucent layer must be made of a material suitable to contact the skin of the face because it covers the entire rear face of the backing layer. By having the translucent layer cover the portion of the collar that would otherwise come into contact with the user's skin, there is greater flexibility to choose a material for the backing layer that may not be as suitable for sensitive skin as the translucent layer. This increases the types of materials that can be used for the backing layer. This may make the mask cheaper to manufacture.

Furthermore, by having the translucent layer cover the rear facing portion of each collar, more light may be diffused to the area of skin contacting the collar via the translucent layer. This may increase the therapeutic effects of the face mask.

The translucent layer may conform to the shape of the backing layer. Specifically, the rear face of the translucent layer 102b may conform to the shape of the rear face of the backing layer 101b. Suitably, the translucent layer adopts the same contours (e.g. shape) as the backing layer. The translucent layer may copy the shape of the mask provided by the rear face of the backing layer such that the contours created by the rear face of the backing layer are maintained once the translucent layer is disposed on the backing layer. Thus, the translucent layer does not interfere with the ability of the mask to conform to the shape of a human face. The translucent layer may be of uniform thickness.

The translucent layer may conform to the shape of the backing layer when comprising the collars, as shown in figure 5. The rear face of the translucent layer conforms to the shape of the exterior lateral surface 7b of each collar. This increases the ease with which the mask may be assembled during manufacture. When the face mask is being manufactured, as will be described in more detail below, the translucent layer and backing layer are formed separately. Because the shape of the rear face of the translucent layer conforms to the shape of the rear face of the backing layer and the exterior lateral surface of each collar, during manufacture, the translucent layer is formed with correspondingly shaped collars to the collars of the backing layer. When attaching the translucent layer to the backing layer to form the face mask, as will be explained in more detail below, the collars of the translucent layer can be attached around the corresponding collars of the backing layer. Thus, having the rear surface of the translucent layer conform to the shape of the exterior lateral surface of each collar provides each layer with alignment features that help ease the assembly of the face mask.

The backing layer may define a step along the periphery of the rear face of the backing layer. The step is not shown in figure 1. An example step 14 is indicated in figure 4. The step may run around the periphery of the rear face of the backing layer. The backing layer may comprise two steps that together form a groove in the backing layer (e.g., an indentation in the backing layer). The groove or indentation may run around the periphery of the rear face of the backing layer. The translucent layer may define a locating feature shaped to mate with the step in the backing layer. For example, the locating feature may be a correspondingly shaped step in the translucent layer that stacks on top of the step in the backing layer. As another example, the locating feature may be a protrusion that mates with the groove or step in the backing layer. The step and locating feature mate together to help secure the translucent layer to the backing layer. The step and locating feature help to align the translucent layer with the backing layer during manufacture.

The translucent layer may cover the peripheral lateral face 101c of the backing layer. In other words, the translucent layer may extend around the peripheral edges of the mask. This may aid with securing the translucent layer to the backing layer during manufacture of the mask.

The mask is generally concave when viewed from its rear side. A frontal plateau of the mask, shown generally at 13 in figure 1, has a gentle concave curvature when viewed from the posterior side of the mask. Peripheral regions of the mask, constituted by cheek regions 4, chin region 5 and forehead region 6 border and adjoin the frontal plateau 13. The peripheral regions are directed more posteriorly, giving the mask generally a bowl shape when viewed from the rear. A nose region 3 is located in the middle of the central plateau. It is located between the eye apertures 10 and above the nose base aperture 11. The nose region is of sharper concavity than the frontal plateau. The lower part of the nose region is broader than the upper part, so as to fit generally to a typical human form.

By way of example only, the depth of parts of the mask may be as follows:
- depth of concavity of frontal plateau: in the range from 10 to 30mm
- height of cheek regions: in the range from 40 to 80mm
- height of chin and/or forehead regions: in the range from 20 to 40mm
- depth of nose region from the frontal plateau: in the range from 20 to 40mm

The mask may comprise attachment structures. For example, at the lateral edges of the mask, the mask constitutes upper (20) and lower (21) tabs. The tabs extend laterally beyond the lateral edge of the cheek regions. Each tab defines a through-hole 22, 23 (e.g., see figure 4). The through-holes can receive straps 15. A strap 15 is shown in figure 4. One strap can pass through and anchor to through-holes 22. A second strap can pass through and anchor to through-holes 23. The straps can pass around the rear of a wearer's head and can be tightened, e.g. by buckles, elastic portions or adjustable hook-and-loop fastenings, to hold the mask in place on the wearer's head. The tabs 20 and 21, which provide fitments for the straps, are spaced apart vertically. Tabs 20 may be located above the majority or the entirety of the vertical extent of the nose region. Tabs 21 may be located below the majority or the entirety of the vertical extent of the nose region. This spacing of the tabs results in the tabs collectively being able to exert a spread rearward force on the sides of the mask, which can assist in conforming the mask to the face of a user over a large vertical extent.

The attachment structures (e.g., tabs 20, 21) are constituted by both the backing layer and the translucent layer. In other words, both the backing layer and translucent layer form the attachment structures.

When a user dons the mask, the pre-formed shape of the mask and the fact that the mask is sufficiently stiff to be self-supporting can mean that the mask can immediately fit generally to the user's face. This induces the wearer to apply the mask in the correct position. It also results in an initial level of conformity of the mask to the user's face.

When the straps are tightened, the flexibility of the mask is such that the action of tightening the straps draws the cheek regions 4 of the mask towards each other generally along the transverse axis of the wearer. Because the attachment structures (e.g., tabs) are constituted by both the translucent layer and backing layer, tightening the straps brings the translucent part of the attachment structure closer to the skin of the wearer. This helps irradiate the skin around the edge of the face (e.g., the hairline), thus improving the therapeutic effect of the mask. If the rearmost parts of the mask are constituted by the translucent layer, they can be expected to be drawn into contact with the wearer's skin when the straps are tightened. This can be advantageous if the translucent layer is formed of a material that is softer and/or of greater biocompatibility than the backing layer.

The action of tightening the straps also draws the frontal plateau of the mask towards the face of the wearer, generally along the sagittal axis of the wearer. The action of tightening the straps may also induce additional curvature on the frontal plateau. These actions all bring the mask into closer conformity with the wearer's face than the initial level of conformity. As the straps are tightened, parts of the mask can move closer to the wearer's face. Thus, an initial level of conformity can be achieved as a result of the mask being able to hold its pre-formed shape. Then an increased level of conformity can be achieved by tightening the straps, with the pre-shaping of the mask inducing at least the cheek regions and the frontal plateau to move, as a result of the pliability of the sheet 1, into greater conformity with the user's face.

As mentioned above, a plurality of light emitters 2 are set into the mask. The light emitters are arranged to emit light rearwardly (i.e., in a rear direction) towards the wearer, so that when the mask is in place on the wearer the skin of the wearer's face can be illuminated or irradiated by the light emitters. This may provide a phototherapeutic effect. For providing a phototherapeutic effect, the light emitters may conveniently emit predominantly or exclusively ultraviolet light. They may emit predominantly or exclusively light in the UV(A) band. They may emit predominantly or exclusively light in the UV(B) band. They may emit predominantly or exclusively light in the near-infrared band. They may emit visible red light. Emissions of such a nature may provide dermatological benefits. Examples of such benefits may include a reduction in conditions of skin irritation or of immune overactivity and/or an improvement in skin tone. Infrared and/or ultraviolet radiation may help to treat acne vulgaris. Infrared radiation may help to treat facial wrinkles as a result of photobiomodulation. The light emitters may emit at a wavelength between 300nm and 1200nm, for example with intensity peaks at one or more of 620 to 640nm or 820 to 840nm or 405 to 425nm. The mask may be capable of emitting with an irradiation intensity over 50% of the posterior area of the mask, measured parallel to the local posterior surface of the mask, of 1 to 60mW/cm², or 10 to 50mW/cm². In one example, the emitters may provide intensity peaks at 405 to 425nm and 820 to 840nm and the irradiation intensity measured as defined above may be not less than 13mW/cm². In another example, the emitters may provide intensity peaks at 405 to 425nm and 620 to 640nm and the irradiation intensity measured as defined above may be not less than 24mW/cm². Some of the light emitters may emit in a first frequency band and others of the light emitters may emit in a second frequency band different from the first frequency band. For example, some of the light emitters may have an emission peak in the region from 400 to 450nm and optionally another emission peak in the range from 600 to 650nm. Others of the light emitters may have an emission peak in the region from 600 to 850nm, or from 800 to 850nm, and also in the region from 400 to 450nm.

The light emitters may, for example, be light-emitting diodes. They may alternatively be fluorescent devices. The light emitters are distributed over the area of the mask, so as to provide a generally uniform illumination to the skin of the wearer. Only a subset of the light emitters are shown in figure 1 for clarity.

Conductive electrical connections extend within the sheet of the mask for supplying power to the light emitters. The electrical connections are connected to a supply cable which is external to the mask. The supply cable can be plugged into a power supply for powering the light emitters. Conveniently the supply cable may terminate in a USB connector. Alternatively, the light emitters may be supplied with power by a battery in the sheet of the mask. A control unit 18 may be coupled by a wired or wireless connection to the light emitters. The control unit may enable a user to perform any of the following actions: to turn on the light emitters, to alter the intensity of the light emitters and to cause the light emitters to extinguish after a predetermined time.

The conductive connections may be sandwiched between the backing layer and the translucent layer. The conductive connections and the light emitters may be provided on a common flexible circuit board. This may make it easier to install them in the mask structure.

As described above, the face mask comprises a backing layer and translucent layer. The layers may be of substantially equal thickness. The translucent layer may have the same or a lower hardness than the backing layer. The translucent layer may be transparent. Each layer may be made of elastomer materials. For example, the layers may be made of a silicone elastomer. The layers may comprise cross-linked polymer. The translucent layer may have a greater degree of cross-linking than the backing layer. The mask may have apertures therethrough, as described above in relation to the nose, mouth and eye apertures. The translucent layer may have the same or a lower elastic modulus as the backing layer. The translucent layer may be more comfortable for a wearer. The translucent layer may be made of a material suited for contacting sensitive skin. The translucent layer may be made of a material that is better suited for contacting sensitive skin than the material forming the backing layer.

The thickness of the backing layer may be in the range from 0.1 to 5.0mm, or from 2.0 to 3.0mm. The thickness of the translucent layer may be in the range from 0.1 to 5.0mm, or from 2.0 to 3.0mm. In some convenient arrangements, the total thickness of the mask (e.g., including both the backing layer and translucent layer) may be in the range from 4.5 to 5.5mm, or in the range from 4.5 to 7.5mm, or in the range from 5.5 to 7.5mm.

The backing layer may be of an elastomeric material, for example a silicone elastomer, or of a rubber, for example a nitrile or butyl rubber. The density of the backing layer may be in the range from 0.5 to 1.5g/cm³, for example in the range from 1.0 to 1.1g/cm³. The Shore A hardness of the backing layer may be in the range from 10 to 100 degrees, for example from 30 to 40 or from 30 to 50 degrees. The backing layer may be formed of Genvan GA9041.

The translucent layer may be of an elastomeric material, for example a silicone elastomer, or of a rubber, for example a nitrile or butyl rubber. It may be of medical grade silicone material. The translucent layer may be formed of Genvan GA9041.

The silicone may be a platinum cure or addition cure silicone. It may comprise platinum as a catalyst. Its principal mode of cross-linking may be through linking of branches off principal polymer chains.

The translucent layer may be softer than the backing layer. The degree of cross-linking in the translucent layer may be less than that of the backing layer. For example, the proportion of cross-linked chains in the translucent layer may be less than 90% or less than 80% that in the backing layer.

The backing layer may be opaque to light. The backing layer may be translucent.

The flexibility of the mask may be such that the force that needs to be applied between the lateral edges of the mask to cause them to touch each other when the mask is bent about a vertical axis is in the range from 0.7 to 1.5N.

The features of the face mask described above may aid the manufacturing process of the face mask, which will be described below.

In a first manufacturing step, the backing layer may be formed. This may be done by injecting silicone monomer or precursor into a mould. The silicone may then be induced to cross-link, for example through the action of heat and/or the presence of one or more cross-linking agents. The cross-linking may be as a result of vulcanisation. To bring about vulcanisation the polymer or precursor may be heated in the presence of sulphur or a sulphur-containing compound suitable for liberating sulphur. The sulphur may form cross-linking bridges between polymer chains. The backing layer may be formed of one continuous piece of polymer material.

In a second manufacturing step, the light emitters may be disposed on the rear face of the backing layer. The electrical interconnections for the light emitters, which may be on a common flexible circuit board, may be applied to the rear face of the backing layer.

In a third manufacturing step, the translucent layer may be formed. The translucent layer is formed separately to the formation of the backing layer. The formation of the translucent layer need not be the third manufacturing step. For example, it could be the first or second manufacturing step. Manufacturing the translucent layer and backing layer may be done concurrently. The translucent layer is formed to cover the entire rear surface of the backing layer. The translucent layer may have the same shape as the backing layer. The translucent layer may comprise similar dimensions to that of the backing layer. The translucent layer may be formed of one continuous piece of polymer material. The translucent layer may be formed by injecting silicone monomer or precursor into a mould, as with the backing layer. That silicone may then be induced to cross-link, for example through the action of heat and/or the presence of one or more cross-linking agents. The cross-linking may be as a result of vulcanisation. If heat is used, it may result in increased cross-linking of the backing layer. The translucent layer may be moulded in a mould not in contact with the backing layer.

In a fourth manufacturing step, the translucent layer is attached to the rear face of the backing layer so as to sandwich the light emitters between the translucent layer and the rear face of the backing layer. Together, the translucent layer and backing layer form the phototherapy mask. Some of the features described above in relation to the face mask may aid the step of attaching the translucent layer to the rear face of the backing layer. For example, where the backing layer defines a step around the periphery of the rear face of the backing layer, and the translucent layer defines a locating feature shaped to mate with the step, attaching the translucent layer to the rear face of the backing layer comprises mating the locating feature with the step. The step feature and the locating feature help align the two layers together.

The eye holes and collars described above may aid in aligning the translucent layer with the backing layer. The eye holes and collars described above may help secure the translucent layer and backing layer together. When considered as a separate piece to the backing layer, the translucent layer also comprises collars around the eye holes. Attaching the translucent layer to the backing layer may comprise sleeving (e.g., attaching) the collars of the translucent layer over the collars of the backing layer. In this way, when the mask has been assembled, the translucent layer conforms to the exterior lateral face of the collars of the backing layer.

The attachment structures may help keep the translucent layer and backing layer together. Each strap that threads through the respective through-hole of the attachment structure must pass through the through-hole formed in both the backing layer and translucent layer. Therefore, when a strap is threaded through the attachment structures, the backing layer and translucent layer are held together by the strap.

An adhesive may be applied to the rear face of the backing layer, and/or the front face of the translucent layer. Attaching the translucent layer to the backing layer may comprise pressing the two layers together to force the adhesive to fill the space between the backing layer and translucent layer.

In the present application the terms "lower", "upper", "front", "forward", "rear", "posterior" etc. are used with reference to the mask in the orientation in which it would be applied to an upright face.

The applicant hereby discloses in isolation each individual feature described herein and any combination of two or more such features, to the extent that such features or combinations are capable of being carried out based on the present specification as a whole in the light of the common general knowledge of a person skilled in the art, irrespective of whether such features or combinations of features solve any problems disclosed herein, and without limitation to the scope of the claims. The applicant indicates that aspects of the present invention may consist of any such individual feature or combination of features. In view of the foregoing description, it will be evident to a person skilled in the art that various modifications may be made within the scope of the invention.

The phrase "configured to" or "arranged to" followed by a term defining a condition or function is used herein to indicate that the object of the phrase is in a state in which it has that condition, or is able to perform that function, without that object being modified or further configured.

## Claims

1. A phototherapy mask (1) comprising:
two eye holes (10);
an opaque backing layer (101) having a rear face (101b) and a front face (101a) and one or more lateral faces (101c) extending between the front face and the rear face, the backing layer comprising a collar (7) around each eye hole, each collar extending rearwardly and each collar having an interior lateral surface (7a) that faces the respective eye hole and an exterior lateral surface (7b);
a translucent layer (102) disposed on the rear face of the backing layer, the translucent layer covering the entire rear face of the backing layer, the translucent layer extending to cover the exterior lateral surface of each collar and a rear facing portion (7c) of each collar; and
a plurality of light emitters (2) sandwiched between the rear face of the backing layer and the translucent layer;
wherein the backing layer and the translucent layer together form a flexible structure that adopts a form that is concave to the rear of the mask.

2. A phototherapy mask as claimed in claim 1, in which the phototherapy mask comprises a nose hole (11) and a mouth hole (12), the translucent layer extending to the edges of the holes such that light from the light emitters can be emitted laterally from the translucent layer at the nose and mouth holes.

3. A phototherapy mask as claimed in any preceding claim, in which the translucent layer has a rear face (102b) and the shape of the rear face of the translucent layer conforms to the shape of the rear face of the backing layer.

4. A phototherapy mask as claimed in any preceding claim, in which the translucent layer has a rear face (102b) and the shape of the rear face of the translucent layer conforms to the shape of the exterior lateral surface of each collar.

5. A phototherapy mask as claimed in any preceding claim, in which the backing layer defines a step (14) along the periphery of the rear face of the backing layer, and the translucent layer defines a locating feature shaped to mate with the step.

6. A phototherapy mask as claimed in any preceding claim, in which the translucent layer covers a peripheral lateral face (101c) of the backing layer.

7. A phototherapy mask as claimed in any preceding claim, in which the phototherapy mask comprises a central plateau (13), cheek regions (4) extending rearwardly and laterally from the central plateau, attachment structures (20, 21) at the rear of the cheek regions, and one or more flexible straps attached to the attachment structures, wherein the attachment structures are constituted by both the backing layer and the translucent layer.

8. A phototherapy mask as claimed in any preceding claim, in which the backing layer and the translucent layer together form a flexible structure that adopts a human facial form.

9. A method for forming a phototherapy mask, comprising:
forming a backing layer of a polymer material, the backing layer having a rear face and a front face and one or more lateral faces extending between the front face and the rear face;
disposing a plurality of light emitters over the rear face of the backing layer;
forming, separately to the formation of the backing layer, a translucent layer of a polymer material for covering the entire rear face of the backing layer; and
attaching the translucent layer to the rear face of the backing layer so as to sandwich the light emitters between the translucent layer and backing layer and form a flexible structure that adopts a form that is concave to the rear of the mask,
wherein the backing layer and translucent layer each comprise two eye holes and collars extending rearwardly around each eye hole, wherein attaching the translucent layer to the rear face of the backing layer comprises fitting each collar of the translucent layer around the corresponding collar of the backing layer.

10. A method as claimed in claim 9, wherein the backing layer defines a step around the periphery of the rear face of the backing layer and the translucent layer defines a locating feature shaped to mate with the step, wherein attaching the translucent layer to the rear face of the backing layer comprises mating the locating feature with the step.

11. A method as claimed in any of claims 9 or 10, wherein the translucent layer is formed as one continuous piece of the polymer material.

12. A phototherapy mask as claimed in any of claims 1 to 8 having a pre-formed three-dimensional shape resembling the contours of a human face.

## Patentansprüche

1. Maske für die Phototherapie (1), umfassend:
zwei Augenlöcher (10);
eine opake Trägerschicht (101) mit einer hinteren Fläche (101b) und einer vorderen Fläche (101a) und einer oder mehreren seitlichen Flächen (101c), die sich zwischen der vorderen Fläche und der hinteren Fläche erstrecken, wobei die Trägerschicht einen Kragen (7) um jedes Augenloch umfasst, wobei sich jeder Kragen nach hinten erstreckt und jeder Kragen eine innere seitliche Fläche (7a), die dem jeweiligen Augenloch zugewandt ist, und eine äußere seitliche Fläche (7b) aufweist;
eine lichtdurchlässige Schicht (102), die auf der hinteren Fläche der Trägerschicht angeordnet ist, wobei die lichtdurchlässige Schicht die gesamte hintere Fläche der Trägerschicht bedeckt, wobei sich die lichtdurchlässige Schicht erstreckt, um die äußere seitliche Fläche jedes Kragens und einen nach hinten weisenden Abschnitt (7c) jedes Kragens zu bedecken; und
eine Vielzahl von Lichtemittern (2), die zwischen der hinteren Fläche der Trägerschicht und der lichtdurchlässigen Schicht eingefügt ist;
wobei die Trägerschicht und die lichtdurchlässige Schicht zusammen eine flexible Struktur bilden, die eine Form annimmt, die konkav zu der Hinterseite der Maske ist.

2. Maske für die Phototherapie nach Anspruch 1, wobei die Maske für die Phototherapie ein Nasenloch (11) und ein Mundloch (12) umfasst, wobei sich die lichtdurchlässige Schicht zu den Kanten der Löcher erstreckt, sodass Licht von den Lichtemittern seitlich von der lichtdurchlässigen Schicht an den Nasen- und Mundlöchern emittiert werden kann.

3. Maske für die Phototherapie nach einem vorhergehenden Anspruch, wobei die lichtdurchlässige Schicht eine hintere Fläche (102b) aufweist und die Form der hinteren Fläche der lichtdurchlässigen Schicht der Form der hinteren Fläche der Trägerschicht entspricht.

4. Maske für die Phototherapie nach einem vorhergehenden Anspruch, wobei die lichtdurchlässige Schicht eine hintere Fläche (102b) aufweist und die Form der hinteren Fläche der lichtdurchlässigen Schicht der Form der äußeren seitlichen Fläche jedes Kragens entspricht.

5. Maske für die Phototherapie nach einem vorhergehenden Anspruch, wobei die Trägerschicht eine Stufe (14) entlang des Umfangs der hinteren Fläche der Trägerschicht definiert und die lichtdurchlässige Schicht ein Lokalisierungsmerkmal definiert, das geformt ist, um sich mit der Stufe zu paaren.

6. Maske für die Phototherapie nach einem vorhergehenden Anspruch, wobei die lichtdurchlässige Schicht eine umfängliche seitliche Fläche (101c) der Trägerschicht bedeckt.

7. Maske für die Phototherapie nach einem vorhergehenden Anspruch, wobei die Maske für die Phototherapie ein zentrales Plateau (13), Wangenregionen (4), die sich nach hinten und seitlich von dem zentralen Plateau erstrecken, Anbringungsstrukturen (20, 21) an der Hinterseite der Wangenregionen und einen oder mehrere flexible Riemen, die an den Anbringungsstrukturen angebracht sind, umfasst, wobei die Anbringungsstrukturen sowohl durch die Trägerschicht als auch die lichtdurchlässige Schicht gebildet sind.

8. Maske für die Phototherapie nach einem vorhergehenden Anspruch, wobei die Trägerschicht und die lichtdurchlässige Schicht zusammen eine flexible Struktur bilden, die eine menschliche Gesichtsform annimmt.

9. Verfahren zum Bilden einer Maske für die Phototherapie, umfassend:
Bilden einer Trägerschicht aus einem Polymermaterial, wobei die Trägerschicht eine hintere Fläche und eine vordere Fläche und eine oder mehrere seitliche Flächen, die sich zwischen der vorderen Fläche und der hinteren Fläche erstrecken, aufweist;
Anordnen einer Vielzahl von Lichtemittern über der hinteren Fläche der Trägerschicht;
Bilden, separat von der Bildung der Trägerschicht, einer lichtdurchlässigen Schicht aus einem Polymermaterial, um die gesamte hintere Fläche der Trägerschicht zu bedecken; und
Anbringen der lichtdurchlässigen Schicht an der hinteren Fläche der Trägerschicht, um die Lichtemitter zwischen der lichtdurchlässigen Schicht und der Trägerschicht einzufügen und eine flexible Struktur zu bilden, die eine Form annimmt, die konkav zu der Hinterseite der Maske ist,
wobei die Trägerschicht und die lichtdurchlässige Schicht jeweils zwei Augenlöcher und Kragen umfassen, die sich nach hinten um jedes Augenloch erstrecken, wobei das Anbringen der lichtdurchlässigen Schicht an der hinteren Fläche der Trägerschicht Anpassen jedes Kragens der lichtdurchlässigen Schicht um den entsprechenden Kragen der Trägerschicht umfasst.

10. Verfahren nach Anspruch 9, wobei die Trägerschicht eine Stufe um den Umfang der hinteren Fläche der Trägerschicht definiert und die lichtdurchlässige Schicht ein Lokalisierungsmerkmal definiert, das geformt ist, um sich mit der Stufe zu paaren, wobei das Anbringen der lichtdurchlässigen Schicht an der hinteren Fläche der Trägerschicht Paaren des Lokalisierungsmerkmals mit der Stufe umfasst.

11. Verfahren nach einem der Ansprüche 9 oder 10, wobei die lichtdurchlässige Schicht als ein kontinuierliches Stück des Polymermaterials gebildet ist.

12. Maske für die Phototherapie nach einem der Ansprüche 1 bis 8 mit einer vorgebildeten dreidimensionalen Form, die den Konturen eines menschlichen Gesichts ähnelt.

## Revendications

1. Masque de photothérapie (1), comprenant :
deux trous oculaires (10) ;
une couche de support opaque (101) comportant une face arrière (101b) et une face avant (101a) et une ou plusieurs faces latérales (101c) s'étendant entre la face avant et la face arrière, la couche de support comprenant un collier (7) autour de chaque trou oculaire, chaque collier s'étendant vers l'arrière et chaque collier comportant une surface latérale intérieure (7a) qui fait face au trou oculaire respectif et une surface latérale extérieure (7b) ;
une couche translucide (102) disposée sur la face arrière de la couche de support, la couche translucide recouvrant toute la face arrière de la couche de support, la couche translucide s'étendant pour recouvrir la surface latérale extérieure de chaque collier et une partie faisant face à l'arrière (7c) de chaque collier ; et
une pluralité d'émetteurs de lumière (2) pris en sandwich entre la face arrière de la couche de support et la couche translucide ;
dans lequel la couche de support et la couche translucide forment ensemble une structure souple qui adopte une forme qui est concave à l'arrière du masque.

2. Masque de photothérapie selon la revendication 1, où le masque de photothérapie comprend un trou nasal (11) et un trou buccal (12), la couche translucide s'étendant jusqu'aux bords des trous de sorte que la lumière à partir des émetteurs de lumière peut être émise latéralement à partir de la couche translucide au niveau des trous nasal et buccal.

3. Masque de photothérapie selon une quelconque revendication précédente, où la couche translucide comporte une face arrière (102b) et le façonnement de la face arrière de la couche translucide épouse le façonnement de la face arrière de la couche de support.

4. Masque de photothérapie selon une quelconque revendication précédente, où la couche translucide comporte une face arrière (102b) et le façonnement de la face arrière de la couche translucide épouse le façonnement de la surface latérale extérieure de chaque collier.

5. Masque de photothérapie selon une quelconque revendication précédente, où la couche de support définit une marche (14) le long de la périphérie de la face arrière de la couche de support, et la couche translucide définit une caractéristique de positionnement façonnée pour s'accoupler avec la marche.

6. Masque de photothérapie selon une quelconque revendication précédente, où la couche translucide recouvre une face latérale périphérique (101c) de la couche de support.

7. Masque de photothérapie selon une quelconque revendication précédente, où le masque de photothérapie comprend un plateau central (13), des régions de joue (4) s'étendant vers l'arrière et latéralement à partir du plateau central, des structures de fixation (20, 21) à l'arrière des régions de joue, et une ou plusieurs sangles souples fixées aux structures de fixation, dans lequel les structures de fixation sont constituées à la fois par la couche de support et la couche translucide.

8. Masque de photothérapie selon une quelconque revendication précédente, où la couche de support et la couche translucide forment ensemble une structure souple qui adopte une forme de visage humain.

9. Procédé de formation d'un masque de photothérapie, comprenant :
la formation d'une couche de support d'un matériau polymère, la couche de support comportant une face arrière et une face avant et une ou plusieurs faces latérales s'étendant entre la face avant et la face arrière ;
la disposition d'une pluralité d'émetteurs de lumière sur la face arrière de la couche de support ;
la formation, séparément de la formation de la couche de support, d'une couche translucide d'un matériau polymère pour recouvrir toute la face arrière de la couche de support ; et
la fixation de la couche translucide à la face arrière de la couche de support de façon à prendre en sandwich les émetteurs de lumière entre la couche translucide et la couche de support et à former une structure souple qui adopte une forme qui est concave à l'arrière du masque,
dans lequel la couche de support et la couche translucide comprennent chacune deux trous oculaires et des colliers s'étendant vers l'arrière autour de chaque trou oculaire, dans lequel la fixation de la couche translucide à la face arrière de la couche de support comprend l'ajustement de chaque collier de la couche translucide autour du collier correspondant de la couche de support.

10. Procédé selon la revendication 9, dans lequel la couche de support définit une marche autour de la périphérie de la face arrière de la couche de support et la couche translucide définit une caractéristique de positionnement façonnée pour s'accoupler avec la marche, dans lequel la fixation de la couche translucide à la face arrière de la couche de support comprend l'accouplement de la caractéristique de positionnement avec la marche.

11. Procédé selon l'une quelconque des revendications 9 ou 10, dans lequel la couche translucide est formée en une seule pièce continue du matériau polymère.

12. Masque de photothérapie selon l'une quelconque des revendications 1 à 8, comportant un façonnement tridimensionnel préformé ressemblant aux contours d'un visage humain.
